**Europäisches Patentamt**

**European Patent Office**

**Office européen des brevets**

(11) Publication number: **0 122 571**
**B1**

(12) **EUROPEAN PATENT SPECIFICATION**

(45) Date of publication of patent specification: **07.01.88**

(21) Application number: **84103901.9**

(22) Date of filing: **07.04.84**

(51) Int. Cl.⁴: **A 61 M 37/02, A 61 B 17/42**

(54) **Device for injecting material directly into uterine cavity.**

(30) Priority: **14.04.83 US 484907**

(43) Date of publication of application:
**24.10.84 Bulletin 84/43**

(45) Publication of the grant of the patent:
**07.01.88 Bulletin 88/01**

(84) Designated Contracting States:
**DE FR GB IT**

(56) References cited:
**GB-A-2 080 112**
**US-A-2 482 622**
**US-A-3 385 300**
**US-A-3 626 928**
**US-A-3 721 229**
**US-A-4 200 097**

(73) Proprietor: **Makler, Amnon**
**50 Disraeli Street**
**Ahuza Haifa 34 334 (IL)**

(72) Inventor: **Makler, Amnon**
**50 Disraeli Street**
**Ahuza Haifa 34 334 (IL)**

(74) Representative: **Hiebsch, Gerhard F., Dipl.-Ing.**
**Erzbergerstrasse 5A Postfach 464**
**D-7700 Singen 1 (DE)**

Courier Press, Leamington Spa, England.

## Description

The present invention relates to a device for injecting a fluid especially a small volume of concentrated spermatozoa, directly into the uterine cavity which device comprises a tubular member for insertion into the cervical canal through which tubular member the fluid may be injected; sealing means communicating with said tubular member for sealing the external orifice of the cervical canal; and a holder supporting said sealing means.

Such a device is known by the US—A—2482622. By this known device carbon dioxide gas is insufflated in this Fallopian tubes for the detection of cancer. There is a tubular member of stainless steel having a brass cannula and a tip of nozzle. The tip is bent to conform the curvature and direction of the cervical canal. The tip is provided with fenestrations for the supply of gas to be injected into the uterus. Slidably on the cannula shaft is a plug or rubber acorn which is used for sealing the opening to the cervical canal after the tip has been introduced therein. An acorn stop is also slidably mounted on the shaft of the cannula behind the sealing member. A set screw is provided in said stop for engagement with said shaft to fixedly position the stop. At the opposite end of the cannula shaft is the inlet opening for connecting the cannula to a source of supply of the liquid or gas to be injected. A valve is used to control the flow of the liquid or gas from a supply line. There is a holder comprising two control members slidably mounted on the outer stainless steel shaft forward of the valve. Said two control members comprise a tenaculum control member having a finger grip for the operator and also comprise a thumb ring limiting or stop member. The entire arrangement is very complicated and large-scale and nonetheless only relatively controllable for the operator. The danger of a harpooning perforation of the uterus still remains, if the acorn stop slipped while occluding the cervix-driving the tip deeper into the uterus than intended by the operator.

By the US—A—3626928 is known an intra uterine washing device having an inlet tube which is attached to an outlet tube. Both tubes being of flexible material. The tip portion of the washer is extended into the uterus until a sealing member, which is adjustable to the size and shape of the uterus, seals the entrance of the uterus by coming into contact and sealing engagement with the walls of the cervix. The sealing member is formed of a material such as rubber and has an acorn shaped configuration. The washing device is in fixed connection with a source of washing fluid contained within a tube and a source of vacuum represented by a syringe.

By the US—A—3385300 is known a cervical cannula comprising a tapered cone made of flexible material, and helical thread means molded onto the cone for easy insertion of the cone into the cervical canal and for retention and sealing of the cone therein. In operation the cone is rotated to advance into the cervical canal by means of the helical thread means. At the posterior end of the cone there is a cannula for admitting fluid, such as gas, contrast liquid or medication, to be injected in the uterus.

It is a principal object of the present invention to provide a device, as known from the US—A—2482622, for injecting a fluid especially a small volume of concentrated spermatozoa, directly into the uterine cavity and allow the fluid to be retained in both the uterine cavity and the cervical canal.

It is a further object of the present invention to provide a device as aforesaid which is safe and effective and is easy to use without trauma which may be caused when using devices which require the use of a tenaculum.

The foregoing objects and advantages may be readily obtained in accordance with the present invention. The device is characterized in that the tubular member is semi-rigid, that, a syringe for containing the fluid to be injected communicates with said tubular member said syringe being held by said holder and that a resilient means engages said holder and urges said sealing means into sealing engagement with the external orifice of the cervical canal.

Special developments of the invention are characterized in the subclaims.

The present invention may be more readily understood from a consideration of the following illustrative drawings in which:

Figure 1 is a side sectional view of the device of the present invention operatively disposed;

Figure 2 is a partial top view of the syringe and holder;

Figure 3 is an enlarged top sectional view showing the tubular member or cannula operatively disposed;

Figures 4, 5, 6 and 7 are sectional views along lines 4-4, 5—5, 6—6 and 7—7 of Figure 3, respectively;

Figure 8 is a top sectional view of the cannula operatively disposed showing approximate actual size; and

Figure 9 is a top sectional view of a foreshortened cannula operatively disposed showing approximate actual size.

As indicated hereinabove, the present invention is useful for injecting any fluid directly into the uterine cavity. However, the present invention is particularly useful for injecting a small volume of concentrated spermatozoa directly into the uterine cavity and therefore the present invention will be discussed hereinafter with regard to this particularly advantageous application.

The major causes of infertility include low sperm concentration by the husband (oligospermia), low rate of sperm mobility (asthenospermia), or both. In certain cases sperm penetration into the wives' cervical canal is impaired by unfavourable cervical mucus.

In most cases semem of oligospermic or asthenospermic men fail to improve by systemic treatment. As an alternative, insemination by concentrated spermatozoa from such specimen is widely used. This treatment includes concentrating the spermatozoa by centrifugation and finally condensing them into a minute volume of about 0.3 cc. In this way the sperm concentration is increased several fold. Following this, the concentrated spermatozoa should be inseminated as close as possible to the location to the location of the ova, that is, injected directly into the uterine cavity or at the least, injected into the cervical canal.

There are several important requirements for effective injection of concentrated spermatozoa. These requirements are admirably met by the device of the present invention. Thus, it should be possible to deposit the available minute volume without losing a significant portion of it within the dead space of the injective system. The device should be provided with a non-traumatic tubular member or cannula that can be penetrated smoothly between the foldings of the cervical canal into the uterine cavity. Such a cannula should be semi-rigid to fit itself to the specific curvature of the uterus, and the device should not require the use of traumatic means such as a tenaculum. The injection system should be provided with an effective and non-traumatic plugging mechanism. This is necessary to prevent back-flow of the injected contents resulting from the tendency of the uterus to contract when irritated by a foreign medium. It is important that the injected contents be retained within the uterine cavity and also have access to the cervical canal for as long as possible. A certain amount of time is necessary for the sperm to get into the Fallopian tubes or be started in the foldings of the cervix from which they can thereafter be released to uterine cavity and the Fallopian tubes.

The device of the present invention effectively achieves all of the foregoing goals. It provides a minimum amount of dead space within the injecting system, e.g., less than about 0.1 cc. It provides a semi-rigid, atraumatic cannula that can be smoothly penetrated into the cervical canal and into the uterine cavity itself without damage to the uterus and without the necessity for the use of a tenaculum. Also, the device of the present invention provides a simple, non-traumatic plugging mechanism to retain sperm in the uterine cavity and cervical canal and to prevent flow of the contents out of the cervix back to the vagina.

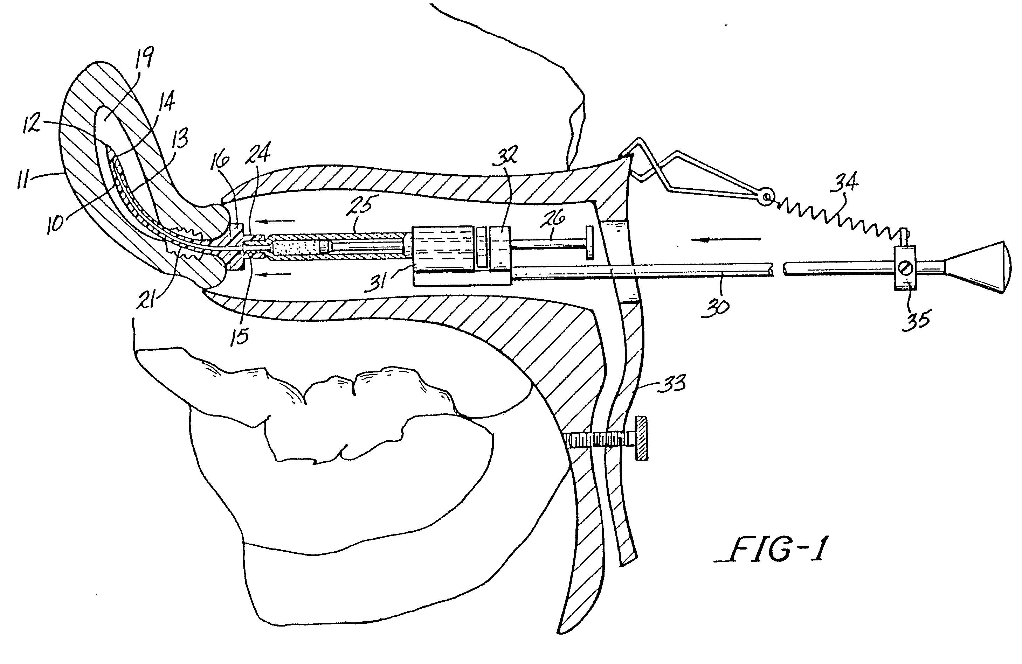

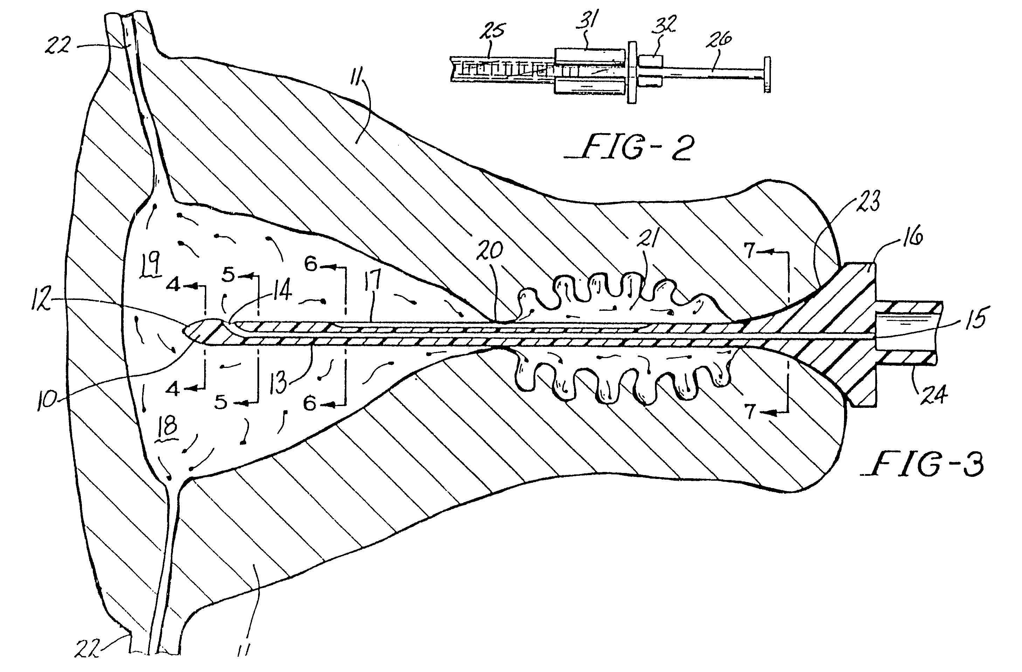

Referring to Figures 1 and 3, the tubular member of cannula 10 is made of semi-rigid material so that it is flexible enough to bend with the natural curvature of the uterus 11 without damaging the uterus as shown in Figure 1 but rigid enough to maintain its shape. Plastic materials such as polyvinyl chloride, polyethylene, polypropylene, polycarbonates and the like are preferred. The cannula is preferably 1.5—2 mm thick and either 12—17 mm long (short version 10' shown in Figure 9) or 40—60 mm long (long version 10 shown in Figures 1, 3 and 8). The short version is intended for intracervical deposition for those women that cannot tolerate intrauterine insemination.

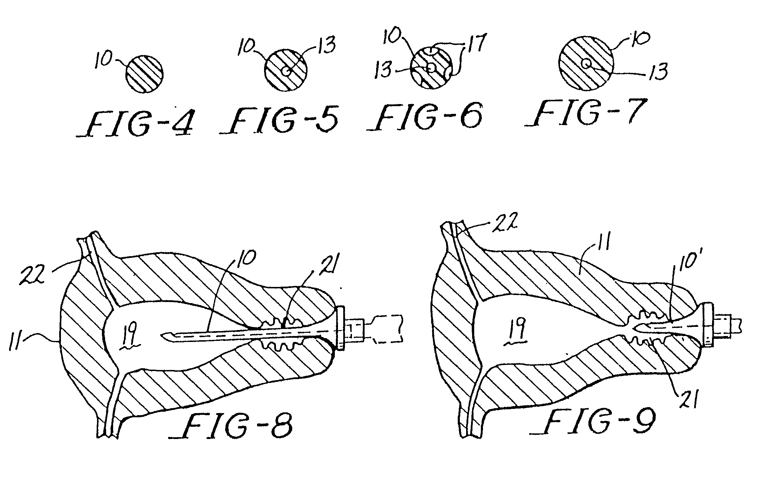

The cannula 10 is provided with a tapered, curved anterior end 12 and a central passageway 13 running the entire length of the cannula with an exit orifice 14 about 1-2 mm behind curved end 12 and an inlet orifice 15 at the enlarged posterior end 16 which serves as sealing means. The outer surface of the cannula 10 is provided with one or more longitudinal grooves 17, with three such grooves 17 being preferred as shown in Figure 6. The longitudinal grooves 17 preferably run from behind exit orifice 14 and terminate before enlarged posterior end 16 to enable sperm 18 to migrate from the uterine cavity 19 through the internal orifice 20 of the cervical canal 21 down to the cervical canal 21 as shown in Figure 3. This feature usefully enables the sperm 18 to contact the cervical canal 21 and pick up cervical fluid which may assist the sperm capacitation in its passage in Fallopian tubes 22. The enlarged posterior end 16 becomes a wide cone blocking the external orifice 23 of the cervical canal 21 having a base of a diameter of about 10—14 mm and terminates in flanges 24 to accept the tip of an ordinary 1 cc elongated tuberculine syringe 25 having conventional plunger 26.

A metal or plastic holder 30 is provided for holding syringe 25 and tubular member or cannula 10 provided with clamp means for holding the syringe firmly in place, such as upstanding flanges 31 and 32 engaging syringe 25 permitting free movement of plunger 26. Holder 30 can, for example, be about 180—220 mm long and about 3—4 mm thick. Adjustable speculum 33 is preferably employed to provide easy access to the vagina. Resilient means, such as spring 34, is preferably employed to urge syringe 25 and enlarged posterior end 16 of tubular member 10 into sealing engagement with the external orifice 23 of the cervical canal 21. Adjustable member 35 is provided on holder 30 and spring 34 is engaged between the speculum 33 and the adjustable member 35 thereby enabling adjustment of the tension. Naturally, other resilient means may be provided that serve the same purpose.

The operation of the device of the present invention is as follows. The tubular member 10 is fixed to syringe 25 by flanges 24 and 0.2—0.3 cc of the concentrated sperm are aspirated into the syringe. The syringe 25 containing sperm and tubular member 10 affixed thereto is placed in clamp 31, 32 of holder 30. Speculum 33 is introduced into the vagina, holder 30, syringe 25 and tubular member 10 inserted therein and the tubular member 10 inserted into the uterine cavity 19 via cervical canal 21 until the external orifice 23 is blocked by the enlarged posterior end of the tubular member 10 as shown in Figure 1. Due to the shape and flexibility of the tubular member, a tenaculum to grasp and pull the uterus to an elongated shape is not required and bleeding and pain are avoided. The tubular member 10 readily follows the natural curvature of the uterus without damaging thereto and without damage to the cervical canal 21 or folding thereof. Spring 34, is fastened with the aid of a clamp on the brim of the speculum 33 and on the adjustable member 35 and the desired tension set by sliding member 35 along holder 30. The contents of syringe 25 are injected through the tubular member 10 into the uterine cavity 19 and circulate throughout the uterine cavity 19 and into the cervical canal 21 via grooves 17. A certain amount of sperm 18 swim towards the Fallopian tubes 22 while the rest can be stored in the foldings of the cervical canal 21 as a reservoir and also mix with the cervical fluid. Sealing means 16 i.e. the enlarged posterior end is pressed firmly against the external orifice 23 of the cervical canal 21 and held in place by resilient means 34. This acts as a retainer and prevents any back-flow of the contents throughout the entire duration of the treatment, i.e., 15—20 minutes, during which time the patient is left alone. Thereafter the device is removed and the patient discharged.

It can be readily seen that the device of the present invention offers many advantages. Thus, the device is non-traumatic and can be readily used without pain or bleeding. There is a virtual absence of dead space. A tenaculum for grasping the uterus is not required. The sealing means 16 need not be inserted into the uterus, i.e., the sealing means 16 effectively seals the uterus by sealing the external orifice 23 of the cervical canal 21. The grooved tubular member or cannula 10 enables access to the cervical canal 21. An alternative device is available for patients who cannot tolerate interuterine injection. The device is simple and easy to use without complications.

**Claims**

1. A device for injecting a fluid, especially a small volume of concentrated spermatozoa, directly into the uterine cavity (19) which device comprises a tubular member (10) for insertion into the cervical canal (21) through which tubular member the fluid may be injected; sealing means (16) communicating with said tubular member (10) for sealing the external orifice (23) of the cervical canal (21); and as holder (30) supporting said sealing means (16), characterized in that the tubular member (10) is semi-rigid, that a syringe (25) for containing the fluid to be injected communicates with said tubular member (10) said syringe (25) being held by said holder (30) and that a·resilient means (34) engages said holder (30) and urges said sealing means (16) into sealing engagement with the external orifice (23) of the cervical canal (21).

2. A device according to claim 1, wherein said resilient means (34) is a spring.

3. A device according to claim 1 or 2, wherein said sealing means (16) is integral with said tubular means (10).

4. A device according to claim 1, 2 or 3 including

a speculum (33) for insertion into the vagina (11) with the holder (30) inserted into the speculum (33) wherein said resilient means (34) is engaged between said holder (30) and speculum (33).

5. A device according to any of claims 1 to 4 wherein said tubular member (10) is suitable for insertion into both the cervical canal (21) and uterine cavity (19).

6. A device according to any of claims 1 to 5 wherein said tubular member (10) includes at least one longitudinal groove (17) terminating before the anterior end (12) thereof to permit access from the uterine cavity (19) to the cervical canal (21).

7. A device according to any of claims 3 to 6 wherein said tubular member (10) has a tapered, curved anterior end (12) and an enlarged posterior end forming said sealing means (16), with a central passageway (13) having an inlet orifice (15) at the posterior end and an exit orifice (14) behind the anterior end (12).

**Patentansprüche**

1. Gerät zum unmittelbaren Injizieren einer Flüssigkeit, insbesondere einer geringen Menge von konzentriertem Spermium, in die Gebärmutter (19), wobei das Gerät ein röhrenförmiges Element (10), durch welches Flüssigkeit injiziert werden kann, zur Einführung in den Gebärmutterhalskanal (21) umfaßt; ferner Dichtungselemente (16), welche mit dem röhrenförmigen Element (10) in Verbindung stehen zur Abdichtung der äußerem Öffnung des Gebärmutterhalskanals (21) sowie eine Halterung (30), welche die Dichtungselemente (16) trägt, dadurch gekennzeichnet, daß das röhrenförmige Element (10) halbstarr ist, daß eine die zu injizierende Flüssigkeit aufnehmende Spritze (25) mit dem röhrenförmigen Element (10) in Verbindung steht, wobei die Spritze (25) von der Halterung (30) gehalten wird, und daß ein elastisches Element (34) mit der Halterung (30) in Eingriff steht sowie das Dichtungselement (16) mit der äußeren Öffnung (23) des Gebärmutterhalskanals (21) in dichtenden Eingriff bringt.

2. Gerät nach Anspruch 1, dadurch gekennzeichnet, daß das elastische Element (34) eine Feder ist.

3. Gerät nach Anspruch 1 oder 2, dadurch gekennzeichnet, daß das Dichtungselement (16) mit dem röhrenförmigen Element (10) eine Einheit bildet.

4. Gerät nach Anspruch 1, 2 oder 3, welches ein Spekulum (33) zur Einführung in die Vagina (11) umfaßt, bei dem die Halterung (30) in das Spekulum eingeführt ist, wobei das elastische Element (34) zwischen der Halterung (30) und dem Spekulum (33) in Eingriff steht.

5. Gerät nach Anspruch 1 bis 4, dadurch gekennzeichnet, daß das röhrenförmige Element (10) zur Einführung sowohl in den Gebärmutterhalskanal (21) als auch in die Gebärmutter (19) ausgebildet ist.

6. Gerät nach einem der Ansprüche 1 bis 5, dadurch gekennzeichnet, daß das röhrenförmige Element (10) zumindest eine längliche Nut (17) aufweist, welche vor dessen vorderen Ende (12) abschließt, um von der Gebärmutter (19) zum Gebärmutterhalskanal (21) Zugang zu gewähren.

7. Gerät nach einem der Ansprüche 3 bis 6, dadurch gekennzeichnet, daß das röhrenförmige Element (10) ein verjüngtes, gebogenes vorderes Ende (12) und ein vergrößertes hinteres Ende, welches das Dichtungselement (16) bildet, aufweist, wobei ein zentraler Durchgang (13) eine Einlaßöffnung (15) am hinteren Ende und eine Auslaßöffnung (14) hinter dem vorderen Ende (12) aufweist.

## Revendications

1. Dispositif pour l'injection directe d'un liquide notamment d'une petite quantité de spermatozoïde concentré dans la cavité utérine (19), étant pourvu d'un élément tubulaire (10) par lequel peut être injecté un liquide et qui est destiné à l'introduction dans le canal cervical (21); en outre d'éléments d'étanchéité (16) qui sont liés à l'élément tubulaire (10) pour l'étanchement de l'orifice extérieur (23) du canal cervical (21); ainsi que d'une fixation (30) portant les éléments d'étanchéité (16), caractérisé en ce que l'élément tubulaire (10) est semi-rigide, qu'une seringue (25) contenant de liquide à injecter est liée à l'élément tubulaire (10), la seringue (25) étant tenue par la fixation (30) et qu'un élément résilient (34) est engrené dans la fixation (30) et met en engrene- ment étanche l'élément d'étanchéité (16) et l'orifice extérieur (23) du canal cervical (21).

2. Dispositif selon la revendication 1 caractérisé en ce que ledit élément résilient (34) est un ressort.

3. Dispositif selon la revendication 1 ou 2 caractérisé en ce que l'élément d'étanchéité (16) forme une unité avec l'élément tubulaire (10).

4. Dispositif selon la revendication 1, 2 ou 3 étant pourvu d'un spéculum (33) destiné à l'introduction dans le vagin (11) avec la fixation (30) introduite dans le spéculum (33), l'élément résilient (34) étant engrené entre la fixation (30) et le spéculum (33).

5. Dispositif selon une des revendications 1 à 4 caractérisé en ce que l'élément tubulaire (10) sert à l'introduction et dans le canal cervical (21) et dans la cavité utérine (19).

6. Dispositif selon une des revendications 1 à 5 caractérisé en ce que l'élément tubulaire (10) possède au moins une rainure longitudinale (17) qui se termine devant l'extrémité avant (12) pour permettre l'accès de la cavité utérine (19) au canal cervical (21).

7. Dispositif selon une des revendications 3 à 6 caractérisé en ce que l'élément tubulaire (10) possède une extrémité avant conique coudé (12) et une extrémité arrière agrandie qui constitue l'élément d'étanchéite (16), un passage central (13) ayant une orifice d'aspiration (15) à son extrémité arrière et une sortie (14) derrière l'extrémité avant (12).

FIG-1

FIG-2

FIG-3

0 122 571

FIG-4     FIG-5     FIG-6     FIG-7

FIG-8

FIG-9

0 122 571